# EUROPEAN PATENT APPLICATION

(11) **EP 4 779 970 A1**
(43) Date of publication of application: **22.07.2026**
(21) Application number: 25152654.7
(22) Date of filing: 17.01.2025
(51) Int. Cl.: H04N 13/117, A61B 1/04, H04N 13/239, H04N 13/243

(54) **DEVICE AND SYSTEM FOR PROVIDING AN IMAGE WITH INFORMATION IN A PRE-DEFINED SPECTRUM AND METHOD THEREFOR**

(71) Applicant: Leica Instruments (Singapore) Pte Ltd, Singapore 618299 (SG)
(72) Inventor: Jung, David, 618299 Singapore (SG)
(74) Representative: Paustian & Partner Patentanwälte mbB

(57) **Abstract**

A first aspect of the present disclosure is related to a device for providing an image with information in a pre-defined spectrum, in particular with fluorescence information, and in particular of a biperspective medical and/or diagnostic imaging device,
configured to:
- obtain a first stereo image, S1-image, from a first perspective on a sample, wherein the S1-image captures light of a first spectrum and comprises a left and a right image as stereo information;
- obtain a second stereo image, S2-image, from the first perspective on the sample, wherein the S2-image captures light in a second spectrum which is different from the first spectrum, in particular fluorescence light, of the sample and comprises a left and a right image as stereo information;
- obtain a third stereo image, A1-image from a second perspective on the sample, wherein the A1-image captures light in a third spectrum that is the same or overlapping with the first spectrum, of the sample and comprises a left and a right image as stereo information;
- determine a fourth image, A2-image, based on one or both of the S1-image and the S2-image and based on the A1-image, wherein the A2-image represents light emitted from the sample and is based on the stereo information of the used images;
- provide an image based on the A2-image, in particular the A2-image, for display.

## Description

### Technical Field

This disclosure is related to devices and systems for providing an image and/or an image stream with information in a pre-defined spectrum, in particular fluorescence information, and for methods therefor.

### Background

A dual-camera setup in an imaging device, e.g. a surgical microscope, provides several key advantages. First, it can offer an operator, e.g. a surgeon, and an assistant individualized, high-quality visual feeds without compromising image clarity or brightness. Each can adjust magnification, focus, or display settings independently, enhancing both comfort and workflow. The assistant's camera allows for simultaneous observation, improving team coordination and communication. Such a setup may however be costly and/or affected by spatial constraints. Improvements are desirable.

### Summary

An object of the present disclosure is to improve imaging of a sample.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a device for providing an image with information in a pre-defined spectrum, in particular with fluorescence information, and in particular of a biperspective medical and/or diagnostic imaging device,
*configured to:*
- *obtain a first stereo image, S1-image, from a first perspective on a sample, wherein the S1-image captures light of a first spectrum and comprises a left and a right image as stereo information;*
- *obtain a second stereo image, S2-image, from the first perspective on the sample, wherein the S2-image captures light in a second spectrum which is different from the first spectrum, in particular fluorescence light, of the sample and comprises a left and a right image as stereo information;*
- *obtain a third stereo image, A1-image from a second perspective on the sample, wherein the A1-image captures light in a third spectrum that is the same or overlapping with the first spectrum, of the sample and comprises a left and* a *right image as stereo information;*
- *determine a fourth image, A2-image, based on one or both of the S1-image and the S2-image and based on the A1-image, wherein the A2-image represents light emitted from the sample and is based on the stereo information of the used images;*
- *provide an image based on the A2-image, in particular the A2-image, for display.*

A device according to the first aspect can be a control device, e.g. a specially programmed computer, configured to control an imaging device and/or it can be a part of the imaging device. A pre-defined spectrum can be defined in the spectral domain and/or in the time domain. A biperspective medical and/or diagnostic imaging device can be a microscope, an exoscope, etc., wherein the biperspective device may comprise a system with at least two cameras and/or is related to two independent cameras. The two cameras are configured to observe a sample from two different perspectives/viewpoints.

A sample can be any object and/or specimen that can be viewed with an imaging device. In a surgical context, a sample can be an organ of a patient and/or a part of an organ. It can be a biopsy, in particular one that is isolated and analyzed in situ. A sample can be or comprise a bone and/or blood of a patient. A sample can also comprise a suture, an incision, or any other artifact placed within or at a being's body for or during a surgical procedure.

An obtaining of an image in the sense of this disclosure can comprise receiving and/or a fetching the image information.

A first stereo image (S1-image) can be provided by a first stereo camera of an imaging system that may be related to a first operator, such as a surgeon. Light of a first spectrum may be related, e.g., to visual light. A first spectrum can comprise all sorts of "non-wavelength-shifted" light, such as reflected light, transmitted light, and intrinsically emitted light (e.g., due to luminescence or incandescence). A first perspective can be based on the viewpoint or mounting point of the first stereo camera.

A stereo camera, such as a camera that captures an S1-image, can be a device that uses two lenses placed some distance apart to simultaneously capture two separate images of the same scene (simple stereo). A stereo camera can also comprise a more complex design beyond two fixed lenses. Such cameras could include multiple lenses or sensor arrays, adjustable interaxial distances, automated alignment systems, synchronized shutters, and/or integrated computational processing.

A second stereo image (S2-image) can come from a second stereo camera that is related to a first operator, such as a surgeon. Light of a second spectrum may be related, e.g., to fluorescence light, e.g. excitation-based fluorescence light and/or autofluorescence light. A stereo fluorescence light camera may be used to capture S2-images, in particular with paired optical paths to capture fluorescence images from different angles. Thereby, a three-dimensional visualization of a, e.g., fluorescently labeled, sample, can be provided. Such a camera may integrate optical filters, specialized detectors, and/or software processing to deliver detailed 3D-fluorescence data. A stereo fluorescence camera may differ from a normal stereo camera by using specialized excitation light, selective filters, and/or sensitive detectors to capture emitted fluorescent signals, rather than relying on ambient illumination and reflectance. Fluorescence light and/or fluorescence spectrum relates to light and/or the range of emitted wavelengths from a fluorescent molecule after excitation by specific light/wavelength. It enables separation of multiple fluorophores/dyes with which a sample is stained and/or autofluorophoric light for detailed, (multi-)color imaging of sample structures and processes within samples.

The S1-image captures a sample from the same perspective (first perspective) as the S2-image. This means that the perspectives are essentially the same, such that the S2-image can be overlaid over the S1-image. For example, by mounting the camera for S1-images next to a camera for S2-images a same (first) perspective is realized for both image types. In case the camera for S1-images and the camera for S2-images are not mounted in the same location or next to each other, the S1 and/or the S2-images may be transformed to represent the sample essentially from the same (first) perspective.

A third stereo image (A1-image) can be provided by a third stereo camera that may be related to a second operator, such as an assistant, who observes the sample. The A1-image represents light from a third spectrum. The third spectrum is different from the second spectrum. The third spectrum can comprise visual light. The third spectrum can overlap and/or be congruent with the first spectrum. For example, the first and the third spectra may be visual light spectra and the second spectrum may comprise one or more fluorescence spectra.

A fourth image (A2-image) may be a single view image or a stereo image. An A2-image may be determined by a device according to the first aspect of this disclosure and/or its determination may be controlled by such a device. The A2-image may have an identical or similar (overlapping) spectrum as the S2-image, e.g. one or more fluorescence spectra. The A2-image is not measured by a distinct camera. Rather, it may be determined based on the information of existing cameras that are configured to either observe different spectra and/or a different perspective. An A2-image may be determined based on either or both of an S1-image or an S2-image. Based on the information comprised by one of these images perspective information can be provided to change the first perspective information of an S2-image at least to be closer to the second perspective (i.e. the perspective of the A1-image). Furthermore, an A2-image is based on the information provided by an A1-image. Based on this information, information can be provided to adapt the first perspective information of the S2-image further to the second perspective, in particular such that the perspective of the A2-image is essentially the same as the second perspective.

After an A2-image is determined it can be provided for display to the second operator. This may include further processing of the A2-image, e.g., merging the A2-image with an A1-image. A providing of an image in the sense of this disclosure can comprise sending the information about the image to one or more receivers, in particular a display, and/or storing the information for others to fetch, e.g. storing the information in a shared memory.

By using first perspective information and second perspective information an image captured only in the first perspective can be transformed to be used for the second perspective. This can be, e.g., helpful if an imaging system, such as a microscope, is equipped with two stereo cameras for a surgeon. One camera captures visual light from a first perspective. The other camera captures fluorescence emissions from (essentially) the same perspective. Furthermore, a third camera for an assistant captures only visual light. This architecture saves costs and needs less installation space. Fluorescence image information from the surgeon's perspective can then be transformed to the assistant's perspective and displayed together with the visual information of the assistant's camera.

An embodiment of the first aspect is related to a device for providing an image with information in a pre-defined spectrum, in particular with fluorescence information,
*wherein the first perspective and the second perspective are located such that a virtual plane defined between the center of the left image and the center of the right image of the S1-image; and which intersects the sample, is also intersected by a line between the left image and the right image of the A1-image.*

A first perspective is defined by the viewpoint of a camera for S1-images and/or by a viewpoint of a camera for S2-images. A second perspective is defined by a viewpoint of a camera for A1-images. The stereo camera(s) for the first perspective may comprise a first baseline, i.e. a straight distance between a left and a right camera. In the same way the stereo camera for the second perspective may comprise a second baseline. The cameras for the first and second perspective may comprise baselines that intersect each other (at least in two of three dimensions). In particular the baselines of the first and/or second camera (for S1-images and S2-images) may intersect the baseline of the third camera (for A1-images) at their center. Furthermore, the intersection may also be at the center of the baseline of the third camera. Furthermore, the baselines may be orthogonal to each other.

The perspectives for S1, S2, and A1 may be aligned such that all views converge on a same point on the sample. The sample can be observed from the first and from the second perspective.

An embodiment of the first aspect is related to a device for providing an image with information in a pre-defined spectrum, in particular with fluorescence information,
*wherein the device is configured to:*
- *determine a first estimated image , in particular as an A2-image, based on the S2-image and with a viewpoint which is located between the viewpoint of the right image and the viewpoint of the left image of the S1-image and*/*or of the S2-image .*

The first estimated image may be a single-view image. The first estimated image comprises information with at least a part of the spectrum of the S2-image. Additionally or alternatively, a first estimated image may be provided as an A2-image itself or be a source for an A2-image. A first estimated image may be an image determined based on the left and the right image of an S1-image. Based on the depth information of the S1-image, a first estimated image may be determined along the baseline (i.e. between the left and the right image) of an S1-image. This can in particular be advantageous if the baseline of an A1-image (i.e. the line between the left and the right image of the A1-image) intersects the baseline of the S1-image. Additionally or alternatively, a first estimated image can also be based on the depth information of the S2-image. However, if the S1-image is a visual light image and the S2-image is a fluorescence light image, the S1-image may be better suited for determining a first estimated image as it may contain more depth information.

An embodiment of the first aspect is related to a device for providing an image with information in a pre-defined spectrum, in particular with fluorescence information,
*wherein the first estimated image is determined based on a disparity map between the right image and the left image of the S1-image and*/*or of the S2-image* .

The first estimated image can be a first estimated image comprising one or more fluorescence spectra.

A disparity map is a two-dimensional representation indicating, e.g. for each pixel in a stereo image, the difference in positions between the left and right views. Each pixel's intensity corresponds to a disparity, allowing recovery of relative depths and enabling three-dimensional understanding of the scene from the two-dimensional left and right images. Based on the disparity (map) pixels for any perspective on the baseline (i.e. that straight line between the left and the right image) can be determined. A disparity map can be determined by mapping the left image of a stereo camera to a right image and another disparity map can be determined by mapping the right image to the left image. Disparity maps can be created for S1-images, S2-images, or for a composite image based on an S1-image and an S2-image (as they capture the sample both from the same perspective). Since, the stereo cameras are aligned vertically, the correspondence problem, i.e. to find corresponding sample pixel of the image of the left camera in the right camera image, can be solved line by line.

Based on the generated disparity maps, a first estimated fluorescence image can then be determined for any point on the baseline between the left image and the right image of the S2-image. In one embodiment, each pixel in an original image may be shifted based on a weighted disparity (disparity value in the same pixel coordinate to a new estimated image. For example, the pixel at location (1,1) has a disparity value of 12, then it can be mapped based on half of the disparity (= 6) to a pixel (1,7) in the new image. This can be done for every pixel in the original image.)

An embodiment of the first aspect is related to a device for providing an image with information in a pre-defined spectrum, in particular with fluorescence information,
*wherein the first estimated image is determined based on an average of a disparity based on a mapping of the left image of the S1-image and*/*or the S2-image in a direction to its respective right image and a disparity based on a mapping of the second image of the S1-image and*/*or the S2-image in a direction to its respective first image*.

By using an average between a disparity for a mapping of a left image to a right image and a disparity for the right image to the left image a more robust disparity map can be determined. Based on this a more robust first estimated image, in particular a first estimated fluorescence image, can be determined.

An embodiment of the first aspect is related to a device for providing an image with information in a pre-defined spectrum, in particular with fluorescence information,
*wherein the first estimated image is based on half of a disparity of the S1-image and*/*or of the S2-image .*

By basing the first estimated (fluorescence) image on half of a disparity of an S1- and/or an S2-image, the first estimated image can be determined as an image from a perspective/viewpoint which is located in the middle of the baseline of an S1- and/or an S2-image. For each pixel of the image the disparity of the respective pixel of the used disparity map can be used.

In case an A1 image is captured with a baseline that intersects the baseline of an S1-image and/or an S2-image in the center, that means the first estimated image is determined from a viewpoint on the baseline of the A1-image. The first estimated image can be provided as A2-image. The first estimated image can then be integrated directly with each of the left and the right image of an A1 image. Alternatively, the first estimated image may be further processed to specifically match the left and the right image of the A1-image.

An embodiment of the first aspect is related to a device for providing an image with information in a pre-defined spectrum, in particular with fluorescence information,
*configured to:*
- *determine a second estimated image , in particular a stereo image, based on the first estimated image*.

The second estimated image can comprise one or more fluorescence spectra.

The first estimated (fluorescence) image can be an image that represents the sample, as a single view image, from a viewpoint between the left image and the right image of an S1-image (or of an S2-image). In case the A1-image is also a stereo image, the first estimated image can be further mapped to each of the left and the right image of the A1-image yielding a second estimated stereo image. This increases the depth perception of the second operator (e.g. the assistant). In particular the second estimated stereo image can be provided as an A2-image or it can be merged with an A1-image to yield a composite A2-image.

An embodiment of the first aspect is related to a device for providing an image with information in a pre-defined spectrum, in particular with fluorescence information,
*wherein the second estimated image is determined based on a disparity map based on the left image and the right image of the A 1-image .*

A second estimated (fluorescence) image can comprise a left image. The left image can be determined for the second perspective, i.e. for the perspective of the left image of the A1-image. The left image may be generated based on disparity map of the A1-image. The mapping based on disparity can be done in the same way as described above. Additionally or alternatively, the second estimated image may comprise a right image. The right image can be determined for the second perspective as well, i.e. for the perspective of the right image of the A1-image. The right image may be generated based on a disparity map of the A1-image.

The first estimated image may be rotated to match the second perspective. For example, if the perspectives are orthogonal to each other, the first estimated image may be rotated by 90 degrees relative to the disparity map(s) of the A1-image. Any further differences in aspect ratio and/or resolution may have been compensated as well.

Once a second estimated stereo image has been determined for the second perspective, it can be provided as an A2-image. Additionally or alternatively the second estimated stereo image can be integrated into an A1-image thereby providing to a second operator an increased depth perception, e.g. for a florescence information, which was only measured for the first perspective.

An embodiment of the first aspect is related to a device for providing an image with information in a pre-defined spectrum, in particular with fluorescence information,
*wherein the second estimated image is based on half of a disparity of the A1-image* .

For each pixel of the image the disparity of the respective pixel of the used disparity map can be used. In particular in case the baseline of an A1-image intersects the baseline of an S1-image such that a first estimated (fluorescence) image is located in the middle of the baseline of the A1-image (at least in two of three dimensions), a mapping of the first estimated image (single view) to the left and the right image of a second estimated stereo image can be based on half the disparity (of the respective point of the disparity map). Then the left and the right image of the second estimated stereo image are determined for the same viewpoint of the left and the right image of the A1-image.

An embodiment of the first aspect is related to a device for providing an image with information in a pre-defined spectrum, in particular with fluorescence information,
*configured to:*
- *determine a composite image based on the A1-image and on the A2-image;*
- *provide the composite image for display.*

An A2-image can be provided as the first estimated (fluorescence) image (single view) or as the second estimated (fluorescence) stereo image. The A2-image can be integrated into the A1 image. This may provide a second operator with an image adapted to her/his viewpoint and integrated into the view of her/his stereo camera.

An embodiment of the first aspect is related to a device for providing an image with information in a pre-defined spectrum, in particular with fluorescence information,
*wherein a baseline of the S1-image and*/*or of the S2-image intersects a*
*baseline of the A1-image in 90 degrees; and*
*configured to:*
   - *determine a rotated S1-image and*/*or a rotated S2-image by 45 degrees; -*
*determine a rotated A1-image by 45 degrees, such that the rotated S1-image and*/*or the rotated S2-image are aligned with their respective sides with the A1-image;* - *determine a third estimated stereo image based on a disparity map between the left image and the right image of the rotated left image and the rotated right image of the S1-image, of the S2-image and*/*or of the A1-image.*

If the left of an S1 and/or an S2-image and a left image of an A1-image are mapped by 45 degrees, then a 'virtual' stereo image pair is created. Therefore, part of the images that are not completely overlapping, e.g. due to the aspect ratios, may be ignored and a direct mapping based on a disparity of the two rotated images can be performed. This may be done from the left image of the S1/S2-image to the right image of the A1-image and from the right image of the S1/S2-image to the left image of the A1 image to add information to occluded areas and improve the result. The third image may be rotated by 45° in the opposite way as the rotated S1/S2-image to match the orientation of the original A1-image. For an output image the third image and the A1-image can be combined.

An embodiment of the first aspect is related to a device for providing an image with information in a pre-defined spectrum, in particular with fluorescence information,
*configured to:*
- *determine one or both of:*
   -- *a plurality of first interpolated images between the left image and the right image of the S1-image and*/*or the S2-image;*
   -- *a plurality of second interpolated images between the left image and the right image of the A1-image and*/*or the A2-image;*
   *- generate a view, e.g. a GIF-image, based on the first generated interpolated images and*/*or a view for the second interpolated images;*
   *- provide the generated views for display.*

Based on the single views and/or on an integrated view a sample can be visualized to a user at a glance. This facilitates an understanding of a sample.

A second aspect of the present disclosure is related to a system for providing an image with information in a pre-defined spectrum, in particular with fluorescence information,
*comprising:*
- *a first stereo camera comprising two cameras for images, S1-images, with light with a first spectrum and which are distanced by a first baseline ;*
- *a second stereo camera comprising two cameras for images, S2-images, with light from a second spectrum, which is different from the first spectrum and which are distanced by a second baseline which is essentially parallel to the first baseline;*
- *a third stereo camera comprising two cameras for images, A1-images, with light for a third spectrum that at least overlaps the first spectrum and which are distanced by a third baseline ;*
*wherein the stereo cameras are arranged such that a two-dimensional projection of the first baseline and of the third baseline intersect, in particular in a common center;*
*configured to:*
- *determine a fourth image , A2-image, based on an S1-image, an S2-image, and an A1-image , wherein the A2-image comprises light emitted in the second spectrum from the sample and from the second perspective;*
- *provide an image based on the A2-image, in particular the A2 image, for display.*

Such a system can have any features and/or functionalities of a device according to the first aspect. A system according to the second aspect of this disclosure enables a fluorescence image for two operators with different perspectives, although fluorescence information is measured only from one perspective. This may save installation space in the optical part of the system and costs for an additional fluorescence camera.

An embodiment of the second aspect is related to a system for providing an image with information in a pre-defined spectrum, in particular with fluorescence information,
*configured to:*
- *display the S1-image and the S2-image as an integrated image; and*/*or*
- *display the A1-image and the A2-image as an integrated image.*

An integrated view for both operators, i.e. for both perspectives may improve common understanding of an observed sample and/or collaboration between the operators.

A third aspect of the present disclosure is related to a method for providing an image with information in a pre-defined spectrum, in particular with fluorescence information,
*comprising the steps:*
- *obtaining a first stereo image, S1-image, from a first perspective on a sample, wherein the S1-image captures light of a first spectrum and comprises a left and a right image as stereo information;*
- *obtaining a second stereo image, S2-image, from the first perspective on the sample, wherein the S2-image captures light in a second spectrum which is different from the first spectrum, in particular fluorescence light, of the sample and comprises a left and a right image as stereo information;*
- *obtaining a third stereo image, S3-image from a second perspective on the sample, wherein the S3-image captures light in a third spectrum that is the same or overlapping with the first spectrum, of the sample and comprises a left and a right image* as *stereo information;*
- *determining a fourth image, S4-image, based on one or both of the S1-image and the S2-image and based on the S3-image, wherein the S4-image comprises light emitted from the sample and is based on the stereo information of the used images;*
- *providing an image based on the S4-image, in particular the S4-image, for display.*

Such a method can have steps related to any features and/or functionalities of a device according to the first aspect and/or a system according to the second aspect of this disclosure.

A further aspect of the present disclosure is related to a computing device comprising a processor configured to carry out the method according to any one of preceding aspects/embodiments.

A further process of the present disclosure is related to a computer program product comprising instructions which, when the program is executed by a computer system, cause the computer system to carry out the method according to any one of the preceding aspects/embodiments.

A further aspect of the present disclosure is related to a computer-readable medium comprising instructions which, when executed by a computer system, cause the computer system to carry out the method according to any one of the preceding aspects/embodiments.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates a device according to an embodiment of this disclosure.
Fig. 2 illustrates a functioning of a device according to an embodiment of this disclosure.
Fig. 3 illustrates a functioning of a device according to an embodiment of this disclosure.
Fig. 4 illustrates a microscope system according to or for embodiments of this disclosure.

Although some aspects have been described in the context of an apparatus (or a system) in the present disclosure, the description of these aspects also represents a description of the corresponding method, where a block or device corresponds to a method step or a feature of a method step.

Analogously, aspects described in the context of a method step also represent a description of a corresponding block, item, or feature of a corresponding apparatus or of a system that may in particular be distributed over different locations and is configured to exchange information between the different locations with respective communication means.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

As used herein the term "and/or" includes any and all combinations of one or more of the associated listed items and may be abbreviated as "/". Expressions as, "for example", "e.g.", or "in particular" denote facultative or optional features that can be combined with all other (mandatory, facultative, or optional) features of the aspects or embodiments of this disclosure, until explicitly stated otherwise.

In the following description reference is made to the accompanying figures which form part of the disclosure and which illustrate specific aspects in which the present disclosure can be understood. Identical reference signs refer to identical or at least functionally or structurally similar features.

### Detailed description

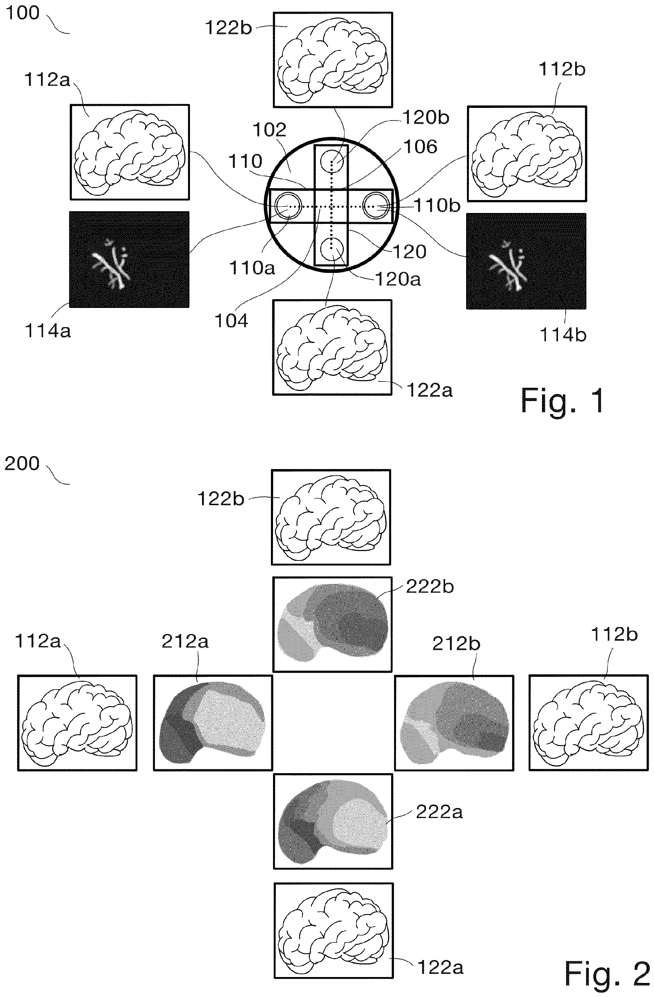

Fig. 1 illustrates a images and an imaging device 100 according to an embodiment of this disclosure. An imaging device 102 comprises a first stereo camera system 110, e.g. for a surgeon. The first stereo camera system 110 comprises a stereo camera for visual light images and a stereo camera for fluorescence light images. Each stereo camera of the system 110 has a left camera 110a and a right camera 110b (the double functionality is indicated by concentric circles). Furthermore, the imaging device 102 comprises a second stereo camera system, e.g. for an assistant. The second stereo camera system comprises a stereo camera for visual light images, only. This can save installation space and costs. The second stereo camera system has also a left camera 120a and a right camera 120b to capture the sample in three dimensions.

The left and the right cameras of the first stereo camera system 110 are arranged such that the distance between them, called baseline 104, is essentially the same. In other words, the visual light stereo camera and the fluorescence light stereo camera are configured to observe a sample from essentially the same viewpoint and/or essentially the same perspective, which is defined by their baseline 104. The left and the right cameras of the second stereo camera system 120 are arranged such that their baseline 106, is perpendicular to the baseline of the first camera system 110. This defines a second perspective, i.e. the perspective of the assistant. Furthermore, the two baselines 104, 106 intersect in their respective center. Hence, two stereo camera system 110, 120 are arranged concentrically with an angle of 90° between each other. Other arrangements are possible.

The first stereo camera system 110 produces four image types from the first perspective: A left visual light image 112a of the sample and a right visual light image 112b of the sample. Additionally, the first stereo camera system produces a left fluorescence light image 114a of the sample and a right fluorescence light image 114b of the sample. By this functionality, e.g., a surgeon, can view the sample in 3D and with visual light and fluorescence light.

The second stereo camera system 120 produces two types of images from the second perspective: A left visual light image 122a of the sample and a right visual light image 122b. By this functionality, e.g., an assistant, can view the sample in 3D and with visual light information. The installation space and the costs for an additional fluorescence image are saved.

Fig. 2 illustrates a functioning of a device, such as the device 102 of Fig. 1 and disparity maps according 200 according to an embodiment of this disclosure. Based on the left image 112a and on the right image 112b of the visual light stereo camera 110 two disparity maps are generated. A first disparity map 212a is generated by mapping the right image 112b to the left image 112a. A second disparity map 212b is determined by mapping the left image 112a to the right image 112b. Furthermore, based on the left (top) image 122a and right image 122b of the second camera system 120 two disparity maps are determined. A first disparity map 222a is generated by mapping the right image 122b to the left image 122a. A second disparity map 222b is determined by mapping the right image 122a to the left image 122b.

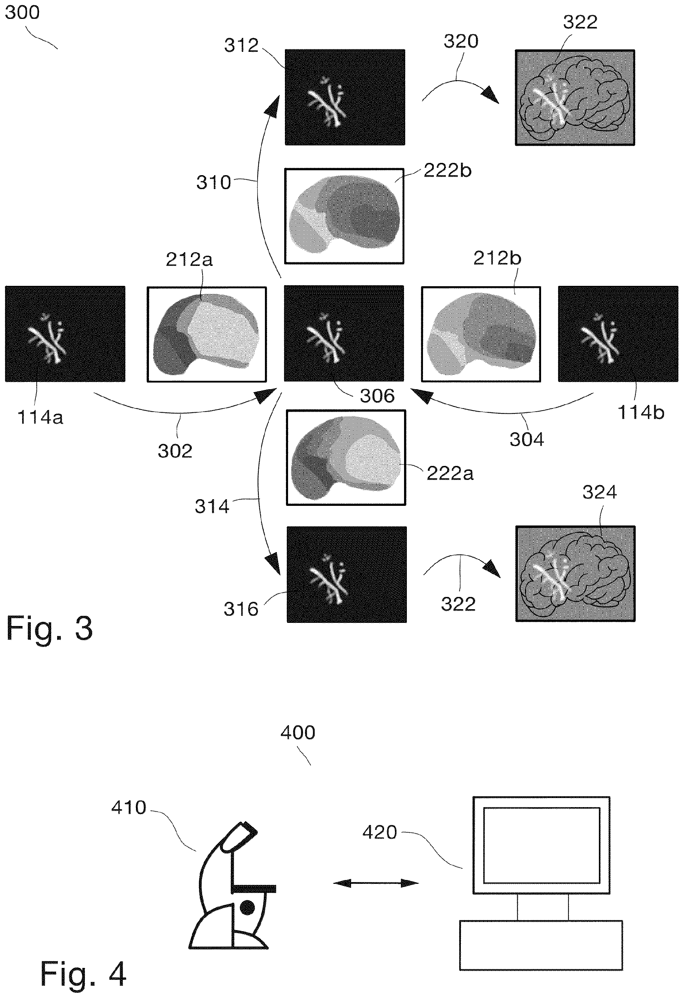

Fig. 3 illustrates a functioning of a device, such as the device 102 of Fig. 1 and the use of disparity maps, such as the disparity maps of Fig. 2, for mapping a fluorescence image observed from a first perspective to a fluorescence image 300 of a second perspective, according to an embodiment of this disclosure.

At first, the fluorescence images 114a and 114b are mapped. The left fluorescence image 114a is mapped based on the disparity map 212a by a first mapping 302. In this case each pixel of the fluorescence image 114a is mapped using half of the disparity (distance) comprised for each pixel in the disparity map 212a in order to arrive at information for the middle image 306. To improve the quality of the middle image, in a second mapping 304 each pixel of the fluorescence image 114b is mapped using half of the disparity (distance) comprised for each pixel in the disparity map 212b in order to arrive at information for the middle image 306. The results of the two mappings 302, 304 are integrated to the middle image 306. The middle image 306 represents a fluorescence image from a perspective that is located at the center of the camera system 102, where the baselines 104 and 106 of both camera systems intersect. The single-view image 306 can already be taken for the second operator, e.g. by integrating it into the stereo images 312, 136. But it can also be further adapted, as described now.

Based on the middle image 306, a stereo view for the second perspective (e.g. the perspective of the assistant) is determined. Therefore, the middle image 306 is mapped 310 based on the disparity map 222b to a right image 312, which shows the sample in the same perspective as the right image 122b. Additionally, the middle image 306 is mapped 314 based on the disparity map 222a to a left image 316, which shows the sample in the same perspective as the left image 122a. The determined left and right fluorescence images 312, 316 constitute a stereo fluorescence image from the second perspective.

Based on the determined left and right fluorescence images 312, 316 a composited image with visual light and fluorescence light can be determined based on the mappings 320, 322 leading to a right composite image 324 and a to left composite image 326. This allows an assistant a composite stereo view with visual light and fluorescence light 3D information just as the surgeon has (but with no extra fluorescence camera for the second perspective).

A further (non-illustrated) aspect of this disclosure is related to a generation of an animated image of a 3D scene, based on the disparity maps. Therefore, disparity maps between stereo pair images can be determined and this information can be used to interpolate views for a smooth transition between a left and a right image of a stereo image pair. The disparity represents an apparent motion of objects between a pair of stereo images and algorithms are readily available to calculate the disparity. Theoretically the left image can be reconstructed from the right image and the disparity map and vice versa. In the same manner intermediate images can be created, by applying only a percentage (each 5%) of the disparity to transform the images. Several intermediate views can be created from a stereo pair and a disparity map of the stereo image. For better results a view may be determined starting from the left image and another view can be determined starting from the right image. The views may then be superimposed and weighted according to the distance of the intended view from the original views.

A method for generating a respective dynamic/animated image, such as a GIF, can comprise the following steps: Record and/or determine a stereo pair. This can be an image pair, e.g. based on the composited images [112a, 114a] and [112b, 114b] or based on the composite images 322, 324. Determine a disparity map for the image pair. Determine intermediated view, e.g. on the baseline 104, 106 between the images of the image pair. Such a determination can be based, e.g., on an interpolation. Finally the image pair and the images in between are combined to an animation such that they can be provided to an operator, e.g. a surgeon.

In order to perform any mappings described in this disclosure, normal image manipulations may be performed, e.g. transforming the images to a same size, etc.

Some embodiments relate to a microscope comprising a system as described in connection with one or more of the Figs. 1 to 3. Alternatively, a microscope may be part of or connected to a system as described in connection with one or more of the Figs. 1 to 3.

Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference signs

- 100: images of imaging device
- 102: imaging device
- 104: baseline
- 106: baseline
- 110: visual light and fluorescence light stereo camera system
- 110a: left visual light and fluorescence surgeon camera system
- 110b: right visual light and fluorescence surgeon camera system
- 112a: left visual light surgeon image
- 112b: left visual light surgeon image
- 114a: left fluorescence surgeon image
- 114b: right fluorescence surgeon image
- 120a: left visual light assistant camera
- 120b: right visual light assistant camera
- 122a: left visual light assistant image
- 122b: right visual light assistant image
- 200: disparity maps
- 212a: disparity map of surgeon camera
- 212b: disparity map of surgeon camera
- 222a: disparity map of assistant camera
- 222b: disparity map of assistant camera
- 300: determination of fluorescence images for assistant
- 302: mapping of left surgeon fluorescence image
- 304: mapping of right surgeon fluorescence image
- 306: middle image
- 310: mapping of middle image
- 312: right assistant image
- 314: mapping of middle image
- 316: left assistant image
- 320: integration of assistant view images
- 322: integration of assistant view images
- 324: right composite assistant image
- 326: left composite assistant image
- 400: system
- 410: microscope
- 420: computer system

## Claims

1. Device for providing an image with information in a pre-defined spectrum, in particular with fluorescence information,
configured to:
- obtain a first stereo image, S1-image, (112a, 112b) from a first perspective (104) on a sample, wherein the S1-image captures light of a first spectrum and comprises a left and a right image as stereo information;
- obtain a second stereo image, S2-image, (114a, 114b) from the first perspective (104) on the sample, wherein the S2-image captures light in a second spectrum which is different from the first spectrum, in particular fluorescence light, of the sample and comprises a left and a right image as stereo information;
- obtain a third stereo image, A1-image (122a, 122b) from a second perspective (106) on the sample, wherein the A1-image captures light in a third spectrum that is the same or overlapping with the first spectrum, of the sample and comprises a left and a right image as stereo information;
- determine a fourth image, A2-image, (306, 312, 316) based on one or both of the S1-image and the S2-image and based on the A1-image, wherein the A2-image represents light emitted from the sample and is based on the stereo information of the used images;
- provide an image based on the A2-image, in particular the A2-image, (322, 324) for display.

2. The device according to the preceding claim,
wherein the first perspective and the second perspective are located such that a virtual plane defined between the center of the left image and the center of the right image of the S1-image; and which intersects the sample, is also intersected by a line between the left image and the right image of the A1-image.

3. The device according to one of the preceding claims;
wherein the device is configured to:
- determine a first estimated image (306), in particular as an A2-image, based on the S2-image (114a, 114b) and with a viewpoint which is located between the viewpoint of the right image and the viewpoint of the left image of the S1-image (112a, 112b) and/or of the S2-image (114a, 114b) .

4. The device according to the preceding claim,
wherein the first estimated image (306) is determined based on a disparity map (212a, 212b) between the right image and the left image of the S1-image (112a, 112b) and/or of the S2-image (114a, 114b).

5. The device according to the preceding claim,
wherein the first estimated image (306) is determined based on an average of a disparity (212a, 212b) based on a mapping of the left image (212a) of the S1-image (112a, 112b) and/or the S2-image in a direction to its respective right image (112b) and a disparity based on a mapping of the second image (112b) of the S1-image and/or the S2-image in a direction to its respective first image (112a).

6. The device according to one of the three preceding claims,
wherein the first estimated image (306) is based on half of a disparity of the S1-image (212a, 212b) and/or of the S2-image (114a, 114b).

7. The device according to one of the preceding claims 3-6,
configured to:
- determine a second estimated image (312, 316), in particular a stereo image, based on the first estimated image (306).

8. The device according to the preceding claim,
wherein the second estimated image (312, 316) is determined based on a disparity map (222a, 222b) based on the left image and the right image of the A1-image (122a, 122b).

9. The device according to the preceding claim,
wherein the second estimated image (312, 316) is based on half of a disparity of the A1-image (122a, 122b).

10. The device according to one of the preceding claims,
configured to:
- determine a composite image based on the A1-image and on the A2-image;
- provide the composite image for display.

11. The device according to one of the preceding claims,
wherein a baseline of the S1-image and/or of the S2-image intersects a baseline of the A1-image in 90 degrees; and
configured to:
- determine a rotated S1-image and/or a rotated S2-image by 45 degrees;
- determine a rotated A1-image by 45 degrees, such that the rotated S1-image and/or the rotated S2-image are aligned with their respective sides with the A1-image;
- determine a third estimated stereo image based on a disparity map between the left image and the right image of the rotated left image and the rotated right image of the S1-image, of the S2-image and/or of the A1-image.

12. The device according to one of the preceding claims,
configured to:
- determine one or both of:
-- a plurality of first interpolated images between the left image and the right image of the S1-image and/or the S2-image;
-- a plurality of second interpolated images between the left image and the right image of the A1-image and/or the A2-image;
- generate a view, e.g. a GIF-image, based on the first generated interpolated images and/or a view for the second interpolated images;
- provide the generated views for display.

13. System for observing samples with information in a pre-defined spectrum, in particular fluorescence information,
comprising:
- a first stereo camera (110a, 110b) comprising two cameras for images, S1-images, with light with a first spectrum and which are distanced by a first baseline (104);
- a second stereo camera (110a, 110b) comprising two cameras for images, S2-images, with light from a second spectrum, which is different from the first spectrum and which are distanced by a second baseline which is essentially parallel to the first baseline;
- a third stereo camera (120a, 120b) comprising two cameras for images, A1-images, with light for a third spectrum that at least overlaps the first spectrum and which are distanced by a third baseline (106);
wherein the stereo cameras are arranged such that a two-dimensional projection of the first baseline (104) and of the third baseline (106) intersect, in particular in a common center;
configured to:
- determine a fourth image (306, 312, 316), A2-image, based on an S1-image, an S2-image, and an A1-image (112a, 112b, 114a, 114b, 122a, 122b), wherein the A2-image comprises light emitted in the second spectrum from the sample and from the second perspective;
- provide an image based on the A2-image, in particular the A2 image, for display.

14. The system according to the preceding claim,
configured to:
- display the S1-image and the S2-image as an integrated image; and/or
- display the A1-image and the A2-image as an integrated image.

15. A method for providing an image with information in a pre-defined spectrum, in particular with fluorescence information,
comprising the steps:
- obtaining a first stereo image, S1-image, (112a, 112b) from a first perspective (104) on a sample, wherein the S1-image captures light of a first spectrum and comprises a left and a right image as stereo information;
- obtaining a second stereo image, S2-image, (114a, 114b) from the first perspective (104) on the sample, wherein the S2-image captures light in a second spectrum which is different from the first spectrum, in particular fluorescence light, of the sample and comprises a left and a right image as stereo information;
- obtaining a third stereo image, S3-image (122a, 122b) from a second perspective (106) on the sample, wherein the S3-image captures light in a third spectrum that is the same or overlapping with the first spectrum, of the sample and comprises a left and a right image as stereo information;
- determining a fourth image, S4-image, based on one or both of the S1-image and the S2-image and based on the S3-image, wherein the S4-image comprises light emitted from the sample and is based on the stereo information of the used images;
- providing an image based on the S4-image, in particular the S4-image, for display.
